(19) Europäisches Patentamt
European Patent Office
Office européen des brevets

(11) Veröffentlichungsnummer: **0 089 517**
**B1**

(12) # EUROPÄISCHE PATENTSCHRIFT

(45) Veröffentlichungstag der Patentschrift: **11.06.86**

(51) Int. Cl.⁴: **C 07 C 93/14**, C 07 C 79/35

(21) Anmeldenummer: **83101949.2**

(22) Anmeldetag: **28.02.83**

(54) **Verfahren zur Herstellung von Diphenyläthern.**

(30) Priorität: **18.03.82 DE 3209878**

(43) Veröffentlichungstag der Anmeldung: **28.09.83 Patentblatt 83/39**

(45) Bekanntmachung des Hinweises auf die Patenterteilung: **11.06.86 Patentblatt 86/24**

(84) Benannte Vertragsstaaten: **AT BE CH DE FR GB IT LI NL**

(56) Entgegenhaltungen:
**EP-A-0 027 555**
**FR-A-2 311 004**

**Die Akte enthält technische Angaben, die nach dem Eingang der Anmeldung eingereicht wurden und die nicht in dieser Patentschrift enthalten sind.**

(73) Patentinhaber: **CELAMERCK GmbH & Co. KG, Binger Strasse 173, D-6507 Ingelheim am Rhein (DE)**

(72) Erfinder: **Sehring, Richard, Dr. Dipl.-Chem., Frankenstrasse 13, D-6507 Ingelheim (DE)**
Erfinder: **Buck, Wolfgang, Dr. Dipl.-Chem., In der Dörrwiese 37, D-6507 Ingelheim (DE)**

**EP 0 089 517 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein verbessertes Verfahren zur Herstellung von Diphenyläthern der allgemeinen Formel

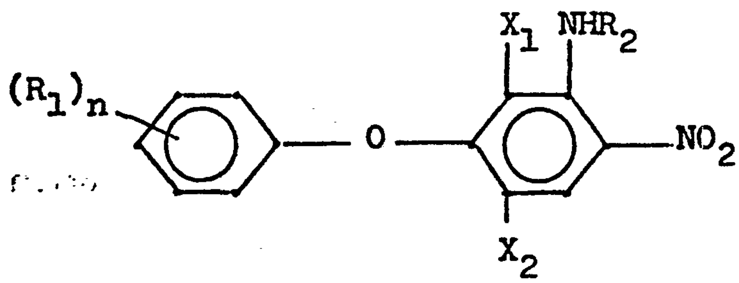

(I)

in der
n eine ganze Zahl von 1 bis 3,
$R_1$ Wasserstoff, Halogen, Alkyl oder Alkoxy mit 1 bis 8 C-Atomen, Trifluormethyl, Acetyl, Cyano,
$R_2$ Wasserstoff; Alkyl, Alkenyl oder Alkinyl mit bis zu 16 C-Atomen; einen durch Hydroxy, $C_1$-$C_4$-Alkoxy, Phenoxy, Halogenphenoxy, $C_1$-$C_4$-Alkylphenoxy, $C_1$-$C_4$-Alkoxy-phenoxy, Nitrophenoxy, Cyanophenoxy, Amino oder $C_1$-$C_4$-Alkylthio substituierten Alkylrest mit 2 bis 6 C-Atomen; gegebenenfalls halogensubstituiertes Benzyl, $NR_3R_4$, $CHR_3$-$COOR_4$ oder $CHR_3$-$CONR_4R_5$ bedeutet, wobei $R_3$, $R_4$ und $R_5$, die gleich oder verschieden sein können, Wasserstoff oder $C_1$-$C_8$-Alkylreste darstellen, und
$X_1$ und $X_2$, die gleich oder verschieden sein können, für Wasserstoff oder Halogen stehen, $X_1$ auch für Methyl.

Die Werte 2 und 3 nimmt n nur an, wenn $R_1$ für Halogen oder Alkyl steht.

Unter "Halogen" sind Fluor, Chlor, Brom und Jod zu verstehen. Bevorzugt sind Fluor, Chlor und Brom, vor allem Fluor und Chlor.

Soweit $R_1$, $R_2$, $R_3$, $R_4$, $R_5$ aliphatische Kohlenwasserstoffreste darstellen oder enthalten, können diese gegebenenfalls auch verzweigt sein. Bevorzugt enthalten diese Reste jedoch 1 bis 2 C-Atome.

Im Rahmen der vorstehenden Definitionen steht $R_1$ bevorzugt für Wasserstoff, Fluor, Chlor, Brom oder Methyl, $R_2$ für Wasserstoff, $C_1$-$C_4$-Alkyl, Allyl, 2-Hydroxyäthyl, 4-Fluor- oder 4-Chlorbenzyl oder Dimethylamino. $R_3$ ist vorzugsweise Wasserstoff oder Methyl, $R_4$ $C_1$-$C_4$-Alkyl. Für $X_1$/$X_2$ sind hauptsächlich die Bedeutungspaare Chlor/Wasserstoff, Methyl/Wasserstoff, Chlor/Brom und Brom/Chlor hervorzuheben. Bedeutet n 2 oder 3, können verschiedene Reste $R_1$ gleichzeitig vorliegen.

Die Verbindungen der allgemeinen Formel I sind z.T. bekannt (DE-OS P 29 38 595). Sie zeichnen sich durch hervorragende herbizide Wirkung aus.

Nach der DE-OS 29 38 595 können die Verbindungen der allgemeinen Formel I erhalten werden, indem man ein Nitrodiphenoxybenzol der allgemeinen Formel

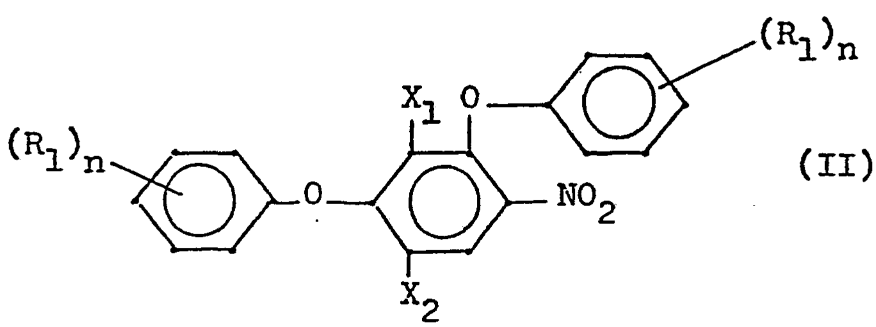

(II),

worin n, $R_1$, $X_1$ und $X_2$ die obige Bedeutung haben, mit einem Amin der Formel $H_2NR_2$ (III) worin $R_2$ die obige Bedeutung hat, bei erhöhter Temperatur umsetzt. Die Verbindungen der Formel II werden hergestellt, indem man eine Verbindung der allgemeinen Formel

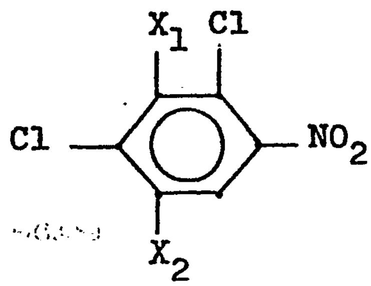

(IV),

worin $X_1$ und $X_2$ wie oben definiert sind, z.B. 2,3,4-Trichlorbenzol, 2,4-Dichlor-3-methylnitrobenzol und 2,3,4,5-Tetrachlornitrobenzol, mit einem entsprechenden Phenolat der allgemeinen Formel

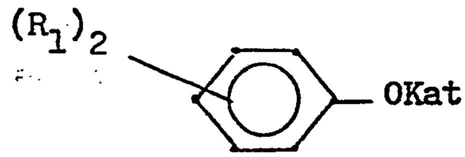

(V)

(Kat gleich 1 Äquivalent eines Kations)
umsetzt. Bevorzugt setzt man die Alkaliphenolate ein, insbesondere das Natriumphenolat, bzw. das Phenol und z.B. ein Alkalicarbonat. Bei einfachen Phenolen, vor allem bei unsubstituiertem Phenol, kann das entsprechende Phenol als Reaktionsmedium dienen. Als besonders günstig wird jedoch die Verwendung von Dimethylsulfoxyd als Lösungsmittel bezeichnet.

Die beiden gegen Phenoxygruppen austauschbaren Chloratome in 2- und 4-Stellung zeigen deutlich abgestufte Reaktionsfähigkeit.

Das bekannte Verfahren ist für die Verwendung im technischen Maßstab weniger geeignet: Bei der Herstellung der Ausgangsstoffe der Formel II kommt es zur Bildung stark gefärbter Nebenprodukte, die eine direkte Weiterverarbeitung erschweren oder unmöglich machen. Störend sind auch phenolische Nebenprodukte, etwa Spaltprodukte des Typs

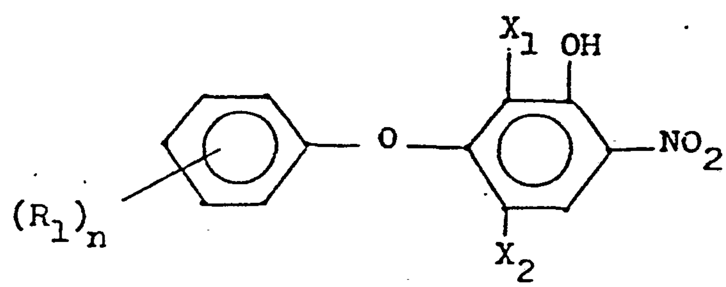

Zur Entfernung der Nebenprodukte ist ein erheblicher Reinigungsaufwand nötig.

Demgegenüber ist aus der französischen Patentanmeldung FR 76 13 943 ein Verfahren zur nucleophilen aromatischen Substitution halogenierter Benzole mit Phenolaten unter Phasentransferbedingungen bekannt bei dem Diphenylether in guter Reinheit entstehen sollen.

Die Umsetzung der Verbindungen der Formel II zu den Endprodukten I nach dem Stand der Technik erfordert z.T. eine Autoklavenreaktion in Lösungsmitteln wie Toluol, Dioxan, chlorierte Kohlenwasserstoffe. Dabei sind im allgemeinen Reaktionszeiten von erheblicher Länge erforderlich (ca. 12 bis 20 Stunden).

Es wurde nun gefunden, daß die Verbindungen der Formel I in höherer Reinheit, in kürzerer Zeit und mit geringerem apparativem Aufwand erhalten werden können, wenn man ein Nitrobenzol der Formel IV heterogen in Gegenwart eines Phasentransferkatalysetors mit einem Phenol der Formel

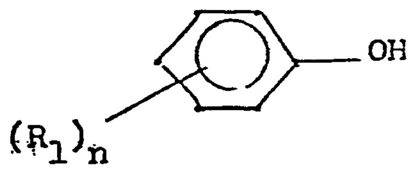

(VI),

worin $R_1$ und n die obige Bedeutung haben, unter Zugabe von wässriger Alkali- oder Erdalkalibase bei erhöhter Temperatur zu der entsprechenden 2,4-Diphenoxyverbindung der Formel II und diese in einem hochsiedenden, Äthergruppierungen enthaltenden Alkohol mit einem Amin der Formel III zu der entsprechenden Verbindung der Formel I umsetzt.

Die erste Reaktionsstufe, die zu den Diphenoxyverbindungen der Formel II führt, kann gewünschtenfalls auch unter Zusatz inerter, mit Wasser nicht mischbarer Lösungsmittel durchgeführt werden. Der entscheidende Vorteil der erfindungsgemäßen Umsetzung besteht in der Verwendung eines üblichen Phasentransferkatalysators. Durch den Zusatz eines solchen Katalysators wird bei Temperaturen zwischen etwa 60 und 150°C, vorzugsweise zwischen 80 und 120, insbesondere 90 - 110°C, ein sehr reines Produkt der Formel II in hervorragender Ausbeute erhalten. Der Phasentransferkatalysator kann praktisch vollständig zurückgewonnen werden. Die Reaktionskomponenten der Formeln IV und VI werden im Molverhältnis 1: 1,8 bis 1: 2,5 eingesetzt. Vorzugsweise verwendet man das Phenol in geringem Überschuß (Molverhältnis 1: 2,1 bis 1: 2,4) und eine der Phenolmenge entsprechende Menge wäßriger Base, vorzugsweise starke Natron- oder Kalilauge. Die Menge des Phasentransferkatalysators kann in weiten Grenzen variiert werden. Als vorteilhaft hat sich erwiesen, etwa 0,005 bis 0,12, vorzugsweise 0,01 bis 0,06 Mol Katalysator pro Mol des Phenols anzuwenden. Geeignete Phasentransferkatalysatoren sind z.B. Triethylbenzylammonium-chlorid oder -bromid; Tetrabutylammoniumchlorid, -bromid,-hydrogensulfat, -hydroxid; Methyltrioctylammoniumchlorid; Tetrabutylphosphoniumbromid. Zahlreiche weitere geeignete Phasentransfer-Katalysatoren sind der Monographie von E.V. Dehmlow und S.S. Dehmlow, "Phase Transfer Catalysis", Verlag Chemie, Weinheim (1980), insbesondere Seite 38 sowie Seite 39 - 43, zu entnehmen.

Die zweite Reaktionsstufe, die Umsetzung der Diphenoxyverbindungen der Formel II zu den Endprodukten der Formel I, gelingt überraschenderweise besonders gut, wenn als Reaktionsmedium Atheralkohole verwendet werden. Geeignete Verbindungen dieser Art sind insbesondere aliphatische oder auch cycloaliphatische Verbindungen, z.B. Diethylenglykol, Triethylenglykol, Dipropylenglykol, Diethylenglykolmonomethyläther, -monoethyläther, -monopropyläther, -monobutyläther, -monobenzyläther, niedere Monoalkyläther des Triethylenglykols oder des Dipropylenglykols, partielle Äther anderer mehrwertiger Alkohole. Die Reaktionstemperatur liegt zwischen ca. 20 und ca. 180°C, vorzugsweise zwischen ca. 40 und 170°C; sie hängt erheblich von dem verwendeten Amin der Formel III ab. Während z.B. Methylamin bei Raumtemperatur umgesetzt werden kann, ist bei der Verwendung von Ammoniak eine höhere Temperatur erforderlich, um die Reaktion innerhalb weniger Stunden zuende zu führen. Eine geringe Erhöhung des Ammoniakdrucks auf 3 - 4 bar kann dazu benutzt werden, um die Umsetzung bei Temperaturen von etwa 120 bis 130°C ohne erhöhten Zeitbedarf durchzuführen.

Die Endprodukte werden in hoher Reinheit und sehr guten Ausbeuten gewonnen.

In den nachstehenden Beispielen wird das Verfahren näher erläutert, ohne daß es auf die beschriebenen Ausführungsformen begrenzt werden soll.

**Herstellung von Diphenoxyverbindungen der Formel II**

**Beispiel (a)**

**2-Chlor-4-nitro-1,3-diphenoxybenzol**

216,2 g (2,3 mol) Phenol, 226,5 g (1 mol) 2,3,4-Trichlornitrobenzol (95,5-prozentig) und 10,0 g Tetrabutylammonium-hydrogensulfat werden in einem Kolben mit Rührer auf 100°C erhitzt. Unter starkem Rühren werden innerhalb von 10 Minuten 187,5 g 49 %ige Natronlauge zuget opft. Das Reaktionsgemisch wird dann 4 Stunden auf ca. 110°C gehalten. Nach dem Abkühlen auf ca. 90°C wird 1 Ltr. Toluol zugegeben und mit Wasser versetzt. Die wäßrige Phase wird abgetrennt. Die Toluolphase wird zweimal mit 100 ml 2 N Salzsäure ausgeschüttelt. Die Toluol-Schicht wird abgetrennt und im Vakuum eingedampft. Der Rückstand besteht nach Gaschromatogramm zu 94,1 % aus der Titelverbindung. Man erhält aus dem Rohprodukt 97,4 % der Theorie an reinem Endprodukt, Fp. 106 - 107°C.

Aus der salzsauren Phase kann der Katalysator mit über 90-prozentiger Ausbeute und genügend rein für die erneute Benutzung in Form des Hydrochlorids zurückgewonnen werden.

**Beispiel (b)**

**3-Methyl-2,4-diphenoxy-nitrobenzol**

20,6 g (0,1 Mol) 2,4-Dichlor-3-methyl-nitrobenzol, 21,6 g (0,225 Mol) Phenol und 9,3 g Tetrabutylphosphoniumchlorid (95-prozentig) (0,03 Mol) werden in einer Rührapparatur zusammengegeben und auf 110°C erhitzt. Unter Rühren tropft man 27 g 50 %ige Natronlauge (0,3 Mol) in das Gemisch und rührt den Ansatz 2 bis 3 Stunden bei Rückflußtemperatur. Nach dem Abkühlen werden 100 ml Toluol und 50 ml Wasser zugesetzt. Das Gemisch wird durchgerührt, anschließend werden die Phasen getrennt. Die Toluollösung wird mit 2 N Salzsäure und anschließend mit 2 N Natronlauge ausgeschüttelt, getrocknet und eingeengt. Man erhält ein braunes Öl (31,7 g = 99 % d.Th. Ausbeute), das beim Verrühren mit wenig Isopropanol kristallisiert. Der gelbliche Feststoff wird abgesaugt und getrocknet, Fp. 55 - 57°C.

Aus der sauren Waschflüssigkeit wird ein Teil des Tetrabutylphosphoniumchlorids zurückgewonnen.

Entsprechend können die nachstehenden Verbindungen erhalten werden.

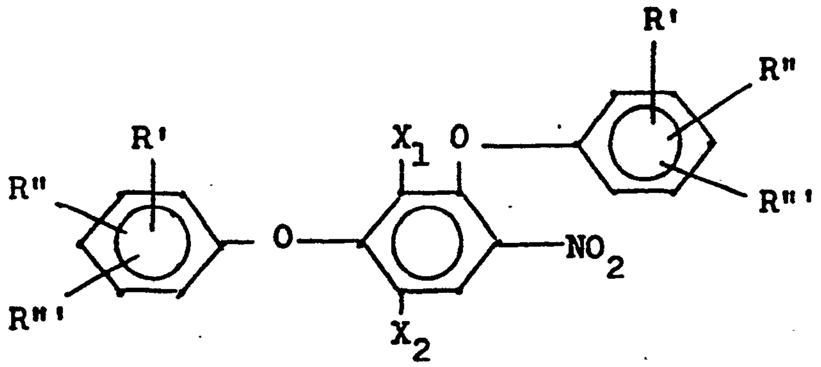

| Beispiel | R″ | R″ | R‴ | $X_1$ | $X_2$ | Fp |
|---|---|---|---|---|---|---|
| 3 | H | H | H | Cl | Cl | 152°C |
| 4 | 4-F | H | H | Cl | H | |
| 5 | 4-$CF_3$ | H | H | Cl | H | |
| 6 | 2-Cl | 4-Cl | H | Cl | H | |
| 7 | 2-Cl | 4-Cl | 5-Cl | Cl | H | |
| 8 | 2-Cl | 4-Br | 5-Cl | Cl | H | |
| 9 | 2-$CH_3$ | 4-Cl | H | Cl | H | |
| 10 | 2-Cl | 4-$SCH_3$ | 5-Cl | Cl | H | |
| 11 | 2-$CH_3$ | 4-$CH_3$ | H | Cl | H | |
| 12 | 2-CN | H | H | Cl | H | |
| 13 | 4-F | H | H | $CH_3$ | H | |
| 14 | 4-$CF_3$ | H | H | $CH_3$ | H | |
| 15 | 2-F | 4-$CH_3$ | H | $CH_3$ | H | |
| 16 | 2-$CH_3$ | 4-$CH_3$ | H | $CH_3$ | H | |
| 17 | 3-Cl | H | H | $CH_3$ | H | |
| 18 | 2-Cl | 5-Cl | H | $CH_3$ | H | |
| 19 | 4-$OCH_3$ | H | H | $CH_3$ | H | |
| 20 | 3-$CF_3$ | H | H | $CH_3$ | H | |

| Beispiel | R' | R" | R"' | $X_1$ | $X_2$ | Fp [°C] |
|---|---|---|---|---|---|---|
| 21 | 4-CN | H | H | $CH_3$ | H | |
| 22 | 4-Br | H | H | $CH_3$ | H | |
| 23 | 4-J | H | H | $CH_3$ | H | |
| 24 | 3-$CH_3$ | 5-$CH_3$ | H | $CH_3$ | H | |
| 25 | 4-$OCH_3$ | H | H | Cl | H | |
| 26 | 2-$CH_3$ | 4-$OCH_3$ | H | Cl | H | |
| 27 | 2-Cl | 4-Br | 5-Cl | Cl | Cl | |
| 28 | 4-$OCH_3$ | H | H | Cl | Cl | |
| 29 | 2-$CH_3$ | 3-$OCH_3$ | 4-$OCH_3$ | Cl | H | |
| 30 | 2-$CH_3$ | 4-Cl | H | Cl | Cl | |
| 31 | 4-$CH(CH_3)_2$ | H | H | Cl | H | |
| 32 | 2-$CH_3$ | 4-$OC_2H_5$ | H | $CH_3$ | H | |
| 33 | 4-$OC_3H_7$ | H | H | Cl | Cl | |
| 34 | 3-$CF_3$ | H | H | Cl | Cl | |
| 35 | 2-Cl | 4-Br | 5-Cl | H | Cl | |
| 36 | 4-Cl | H | H | Cl | H | |
| 37 | 4-Cl | H | H | $CH_3$ | H | |
| 38 | 3-Cl | 5-Cl | H | $CH_3$ | H | |
| 39 | 2-F | H | H | $CH_3$ | H | |
| 40 | 2-CN | H | H | $CH_3$ | H | |
| 41 | 2-$CH_3$ | H | H | $CH_3$ | H | |
| 42 | 3-$CH_3$ | H | H | $CH_3$ | H | |

**Herstellung der Endprodukte der Formel I**

**Beispiel 1**
**2-Chlor-3-phenoxy-6-nitroanilin**

173 g 2-Chlor-4-nitro-1,3-diphenoxybenzol (Rohprodukt nach Beispiel (a)) werden in 500 ml Triethylenglykol gelöst. Bei 165°C wird 6 Stunden lang Ammoniak durch die Lösung geleitet. Das Dünnschichtchromatogramm zeigt dann kein Ausgangsprodukt mehr. Unter Kühlung mit Eis werden 250 ml 2 N Natronlauge und 300 ml Wasser zugegeben. Das Reaktionsprodukt fällt aus und wird bgesaugt. Man erhält 95 % der Theorie an Rohprodukt, as nach Gaschromatogramm 95,8 % Titelverbindung enthält. Die Gesamtausbeute über beide Stufen beträgt 95,3 % d.Th. Fp. 81-82oC (aus Isopropylalkohol).

Zum gleichen Ergebnis gelangt man bei Verwendung von Diethylenglykol bzw. Diethylenglykolmonobutyläther als Lösungsmittel anstelle von Triethylenglykol.

Bei weniger flüchtigen bzw. leichter reagierenden Aminen genügt es, die Komponenten bei Temperaturen zwischen Raumtemperatur und ca. 160°C reagieren zu lassen.

**Beispiel 2**
2-Methyl-6-nitro-3-phenoxy-N-methylanilin

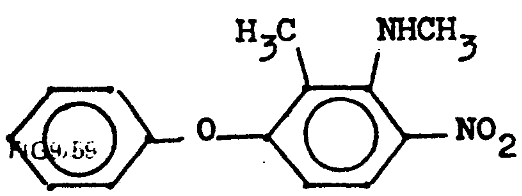

32,1 g 3-Methyl-2,4-diphenoxy-nitrobenzol (0,1 Mol) werden in 100 ml Diäthylenglykol gelöst. Unter Rühren bei 100°C wird Methylamin langsam in die Lösung geleitet. Der Fortgang der Reaktion wird dünnschichtchromatographisch verfolgt (DC-Fertigplatten (MERCK/Darmstadt) Kieselgel 60 $F_{254}$, Toluol/Heptan 1: 1)).

Nach 3 1/2 Stunden ist die Umsetzung beendet. Nach Abkühlen wird das Produkt durch Zugabe von 2 N Natronlauge unter Anreiben ausgefällt, abgesaugt und mit Wasser nachgewaschen. Gelber Feststoff; Fp. 62°C.

Ausbeute: 23 g (89,2 % d.Th.)

Entsprechend erhält mandie nachstehenden Verbindungen

**Tabelle I**

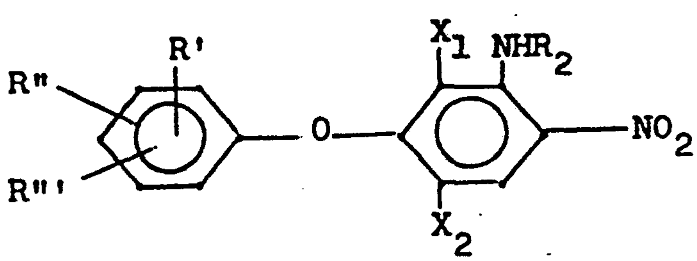

| Beispiel | R' | R" | R"' | $X_1$ | $X_2$ | $R_2$ | Fp [°C] |
|---|---|---|---|---|---|---|---|
| 2 | H | H | H | Cl | Cl | H | |
| 3 | H | H | H | Cl | H | $CH_2CH_2$ - 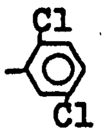 | Öl |
| 4 | H | H | H | H | Cl | $CH_2=CH-CH_2$ | 81-82 |
| 5 | H | H | H | H | Cl | $CH_2-CH_2NH_2 \cdot HCl$ | 255 (Zers.) |
| 6 | H | H | H | Cl | H | H | 81-83 |
| ~~7~~ | ~~2-Cl~~ | ~~4-$SCH_3$~~ | ~~5-Cl~~ | ~~Cl~~ | ~~H~~ | ~~$CH_2-C_6H_5$~~ | ~~Öl~~ |
| 8 | 2-Cl | 4-Br | 5-Cl | H | Cl | $CH_2=CH-CH_2$ | 103 |
| 9 | 2-Cl | 4-Br | 5-Cl | Cl | H | H | |
| 10 | 2-Cl | 4-Br | 5-Cl | H | Cl | $CH_2-C_6H_5$ | 157 |
| ~~11~~ | ~~2-Cl~~ | ~~4-$SCH_3$~~ | ~~5-Cl~~ | ~~Cl~~ | ~~H~~ | ~~$CH_2=CH-CH_2$~~ | ~~107~~ |

| Beispiel | R' | R" | R'" | $X_1$ | $X_2$ | $R_2$ | Fp [°C] |
|---|---|---|---|---|---|---|---|
| 12 | H | H | H | Cl | H | 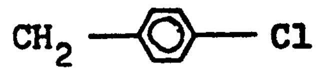 | 95-96 |
| 13 | H | H | H | Cl | H | 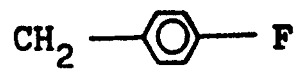 | 75-76 |
| ~~14~~ | ~~H~~ | ~~H~~ | ~~H~~ | ~~Cl~~ | ~~H~~ | 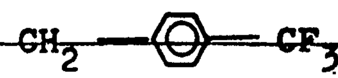 | Öl |
| ~~15~~ | ~~H~~ | ~~H~~ | ~~H~~ | ~~H~~ | ~~Cl~~ | 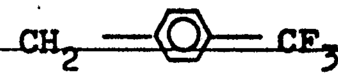 | 140-142 |
| 16 | 4-CN | H | H | Cl | H | $CH_2=CH-CH_2$ | 85-87 |
| 17 | H | H | H | Cl | H | $N(CH_3)_2$ | Öl |
| 18 | H | H | H | Cl | H | $-(CH_2)_6-NH_2$ | Öl |
| 19 | 4-F | H | H | Cl | H | $C_2H_5$ | 69-70 |
| 20 | $4-OCH_3$ | H | H | Cl | H | $CH_3$ | 100-101 |
| 21 | H | H | H | Cl | H | $CH_3$ | 72-74 |
| 22 | 4-Cl | H | H | Cl | H | $CH_3$ | 71-72 |
| 23 | H | H | H | Cl | Cl | H | |
| 24 | $4-CF_3$ | H | H | Cl | H | H | |
| 25 | 2-Cl | 4-Cl | H | Cl | H | H | |

| Beispiel | R' | R" | R"' | $X_1$ | $X_2$ | $R_2$ | Fp [°C] |
|---|---|---|---|---|---|---|---|
| 26 | 2-CN | H | H | Cl | H | H | |
| 27 | 4-$OCH_3$ | H | H | Cl | H | H | |
| 28 | 2-$CH_3$ | 4-$OCH_3$ | H | Cl | H | H | |
| 29 | 2-Cl | 4-Br | 5-Cl | Cl | Cl | H | |
| 30 | 4-$OCH_3$ | H | H | Cl | Cl | H | |
| 31 | 2-$CH_3$ | 3-$OCH_3$ | 4-$OCH_3$ | Cl | H | H | |
| 32 | 2-$CH_3$ | 4-Cl | H | Cl | Cl | H | |
| 33 | 4-$CH(CH_3)_2$ | H | H | Cl | H | H | |
| 34 | 4-$OC_3H_7$ | H | H | Cl | Cl | H | |
| 35 | 3-$CF_3$ | H | H | Cl | Cl | H | |
| 36 | H | H | H | $CH_3$ | H | H | 76-78 |
| 37 | H | H | H | $CH_3$ | H | $CH_2$=CH-$CH_2$ | RF 0,448* |
| 38 | 4-F | H | H | $CH_3$ | H | H | 93-95 |
| 39 | 4-Cl | H | H | $CH_3$ | H | H | 95-97 |
| 40 | 2-Cl | 5-Cl | H | $CH_3$ | H | H | 88-90 |
| 41 | 3-Cl | 5-Cl | H | $CH_3$ | H | H | 122-123 |
| 42 | 4-$CH_3$ | H | H | $CH_3$ | H | H | 88-90 |

| Beispiel | R' | R" | R"' | $X_1$ | $X_2$ | $R_2$ | Fp [°C] |
|---|---|---|---|---|---|---|---|
| 43 | 4-$OCH_3$ | H | H | $CH_3$ | H | H | 101-104 |
| 44 | 2-Cl | H | H | $CH_3$ | H | H | RF 0,262* |
| 45 | 3-Cl | H | H | $CH_3$ | H | H | 93-95 |
| 46 | 2-F | H | H | $CH_3$ | H | H | 96-98 |
| 47 | 2-CN | H | H | $CH_3$ | H | H | 150-154 |
| 48 | 2-$CH_3$ | H | H | $CH_3$ | H | H | braunes Öl |
| 49 | 3-$CH_3$ | H | H | $CH_3$ | H | H | 84-88 |
| 50 | 3-$CF_3$ | H | H | $CH_3$ | H | H | 81-83 |
| 51 | 4-CN | H | H | $CH_3$ | H | H | 213-215 |
| 52 | H | H | H | $CH_3$ | H | $C_2H_5$ | RF 0,392* |
| 53 | 4-Br | H | H | $CH_3$ | H | H | 79-81 |
| 54 | 4-J | H | H | $CH_3$ | H | H | RF 0,215* |
| 55 | 3-$CH_3$ | 5-$CH_3$ | H | $CH_3$ | H | H | |
| 56 | 2-$CH_3$ | 4-$OC_2H_5$ | H | $CH_3$ | H | H | |
| 57 | H | H | H | $CH_3$ | H | $CH_3$ | RF 0,352* |
| 58 | 4-$CH_3$ | H | H | $CH_3$ | H | n-$C_3H_7$ | RF 0,456* |
| 59 | H | H | H | $CH_3$ | H | $CH(CH_3)_2$ | RF 0,394* |

| Beispiel | R' | R" | R"' | $X_1$ | $X_2$ | $R_2$ | Fp [°C] |
|---|---|---|---|---|---|---|---|
| 60 | H | H | H | $CH_3$ | H | $n-C_4H_9$ | RF 0,503* |
| 61 | H | H | H | $CH_3$ | H | $CH_2CH(CH_3)_2$ | RF 0,480** |
| 62 | H | H | H | $CH_3$ | H | $C_2H_5$ | RF 0,392* |
| 63 | 4-F | H | H | $CH_3$ | H | $C_2H_5$ | RF 0,428* |
| 64 | 4-F | H | H | $CH_3$ | H | $n-C_3H_7$ | RF 0,461* |
| 65 | $2-OCH_3$ | H | H | $CH_3$ | H | H | 100-102 |
| 66 | $3-CH_3$ | $5-CH_3$ | H | $CH_3$ | H | $CH_2=CH-CH_2$ | RF 0,441* |
| 67 | $4-CH_3$ | H | H | $CH_3$ | H | $CH_3$ | RF 0,3438* |
| 68 | 4-F | H | H | $CH_3$ | H | $CH(CH_3)_2$ | RF 0,407* |
| 69 | 4-F | H | H | $CH_3$ | H | $n-C_4H_9$ | RF 0,524* |
| 70 | $4-CH_3$ | H | H | $CH_3$ | H | $n-C_4H_9$ | RF 0,514* |
| 71 | 4-F | H | H | $CH_3$ | H | $CH_3$ | 60-63 |
| 72 | $4-CH_3$ | H | H | $CH_3$ | H | $CH_3$ | RF 0,344* |
| 73 | $4-CH_3$ | H | H | $CH_3$ | H | $CH(CH_3)_2$ | RF 0,440* |
| 74 | $4-CH_3$ | H | H | $CH_3$ | H | $C_2H_5$ | RF 0,432* |
| 75 | $2-CH_3$ | H | H | $CH_3$ | H | $C_2H_5$ | RF 0,357 |

* RF-Werte: Bestimmt auf DC-Fertigplatten Kieselgel 60 $F_{254}$ (MERCK/Darmstadt); Laufmittel Toluol, Temperatur 24°C.

** wie vorstehend, jedoch Laufmittel Toluol/Hexan 1 : 1, 23°C.

**Tabelle II**
Verbindungen der Formel

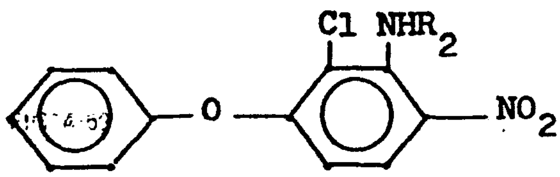

(Die Verbindungen werden als Öle erhalten)

| Nr. | $R_2$ | RF-Werte 27°C Aceton/Heptan 1 : 1 | Toluol |
|---|---|---|---|
| 1 | $C_2H_5$- | 0,51 | 0,46 |
| 2 | n-$C_3H_7$ | 0,615 | 0,47 |
| 3 | i-$C_3H_7$ | 0,61 | 0,45 |
| 4 | n-$C_4H_9$ | 0,625 | 0,50 |
| 5 | i-$C_4H_9$ | 0,60 | 0,51 |
| 6 | HO-$CH_2$-$CH_2$ | 0,445 | 0,01 |
| 7 | -CH($C_2H_5$)$_2$ | 0,47* | |
| 8 | -$CH_2$-C≡CH | 0,31 | |

* = Toluol/Cyclohexan 1 : 1

**Tabelle III**
Verbindungen der Formel

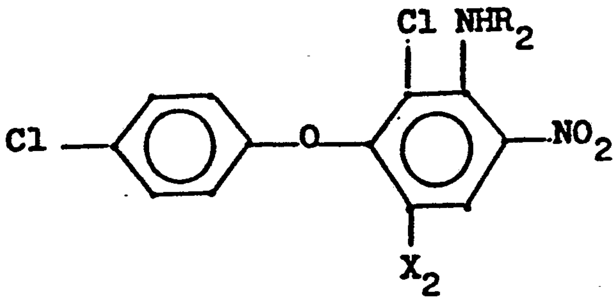

| Nr. | $R_2$ | $X_2$ | Eigenschaften/Fp. |
|---|---|---|---|
| 1 | $CH_3$ | Cl | rote Kristalle Fp. 101°C |
| 2 | $C_2H_5$ | Cl | hellbraunes Öl, dünnschichtchromatographisch einheitlich |
| 3 | $i\text{-}C_3H_7$ | Cl | orange Kristalle Fp. 83°C. |
| 4 | $n\text{-}C_4H_9$ | Cl | oranges Öl, dünnschicht-chromatograph. einheitlich |
| 5 | $n\text{-}C_{12}H_{25}$ | Cl | orange Kristalle Fp. 43°C |
| 6 | $n\text{-}C_{18}H_{37}$ | Cl | 47—48°C |
| 7 | $-CH_2-CH_2-OH$ | Cl | dunkles Öl |
| 8 | $-CH(C_2H_5)_2$ | Cl | |
| 9 | $-CH=CH-CH_2$ | H | RF 0,51 (DC Fertigplatten, Kieselgel 60 $F_{254}$, Laufmittel Toluol, 27°C |
| 10 | $-CH(C_2H_5)_2$ | H | |

**Tabelle IV**

Aus 1,3-Diphenoxybenzolen der Formel

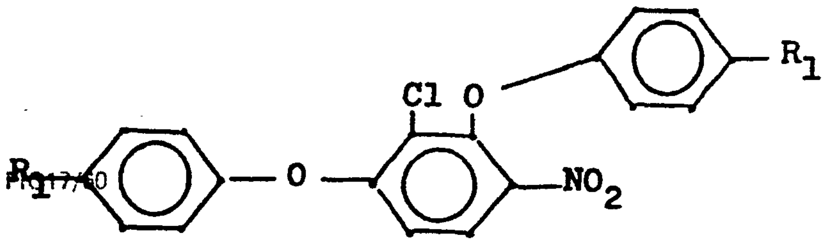

in denen $R_1$ die unten angegebene Bedeutung hat werden die nachstehenden Verbindungen der Formel

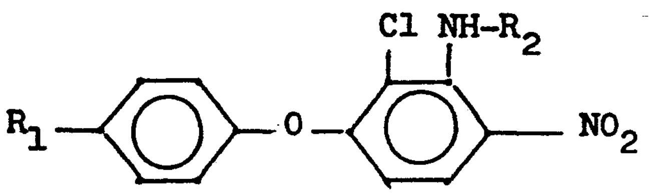

| Nr. | $R_1$ | $R_2$ | RF-Werte 27°C Aceton + Heptan 1 : 1 | RF-Werte 27°C Toluol |
|---|---|---|---|---|
| 1 | $CH_3$ | $CH_3$ | 0,61 | 0,45 |
| 2 | $CH_3$ | $CH_2{=}CH$ $CH_2$ | 0,63 | 0,515 |
| 3 | $OCH_3$ | $n\text{-}C_3H_7$ | 0,59 | 0,29 |

**Patentansprüche**

1. Verfahren zur Herstellung von Verbindungen der Formel

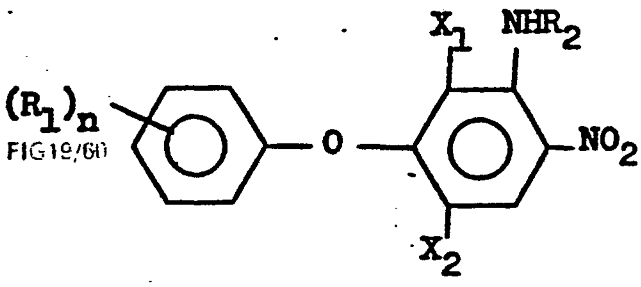

(I),

in der
n eine ganze Zahl von 1 bis 3, darstellt, wenn
$R_1$ für Wasserstoff Halogen oder Alkyl mit 1 bis 8 C-Atomen steht
oder
n 1 darstellt, wenn
$R_1$ für Wasserstoff, Trifluormethyl, Alkoxy mit 1 bis 8-C-Atomen oder Acetyl steht,
$R_2$ Wasserstoff; Alkyl, Alkenyl oder Alkinyl mit bis zu 16 C-Atomen; einen durch Hydroxy, $C_1$-$C_4$-Alkoxy, Phenoxy, Halogenphenoxy, $C_1$-$C_4$-Alkylphenoxy, $C_1$-$C_4$-Alkoxy-phenoxy, Nitrophenoxy, Cyanophenoxy, Amino oder $C_1$-$C_4$-Alkylthio substituierten Alkylrest mit 2 bis 6 C-Atomen; gegebenenfalls halogensubstituiertes Benzyl, $NR_3R_4$, $CHR_3$-$COOR_4$ oder $CHR_3$-$CONR_4R_5$ bedeutet, wobei $R_3$, $R_4$ und $R_5$, die gleich oder verschieden sein können, Wasserstoff oder $C_1$-$C_8$-Alkylrest darstellen, und
$X_1$ und $X_2$, die gleich oder verschieden sein können, für Wasserstoff oder Halogen stehen, $X_1$ auch für Methyl, bei dem in der ersten Stufe

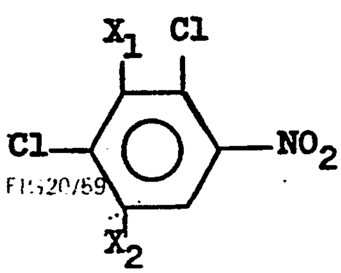

(IV),

in dem $X_1$ und $X_2$ die obige Bedeutung haben, mit einem Phenol der Formel

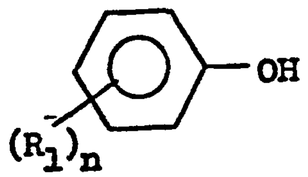

(VI),

worin n und $R_1$ die obige Bedeutung haben, in Gegenwart einer wäßrigen Alkali- oder Erdalkalibase umgesetzt wird und in zweiter Stufe die erhaltene Verbindung der Formel

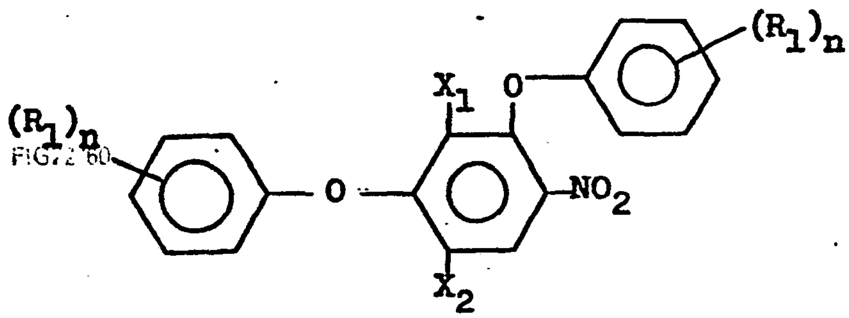

(II),

in der n, $R_1$ $X_1$ und $X_2$ die obige Bedeutung haben, mit einem Amin der Formel
$H_2NR_2$ (III),
in der $R_2$ wie oben definiert ist, zur Reaktion gebracht wird, dadurch gekennzeichnet, daß die erste Stufe der Umsetzung in Gegenwart eines üblichen Phasentransferkatalysators erfolgt und daß für die zweite Stufe als Lösungsmittel ein Äthergruppierungen enthaltender Alkohol verwendet wird.

2. Verfahren nach Anspruch 1, dadurch gekennzeichnet, daß in der 1. Stufe als Nitrobenzol der Formel IV 2,3,4-Trichlornitrobenzol, als Phenol der Formel VI Phenol oder 4-Fluorphenol und in der 2. Stufe als Amin der Formel III Ammoniak oder Methylamin verwendet wird.

3. Verfahren nach Anspruch 1, dadurch gekennzeichnet, daß in der 1. Stufe als Nitrobenzol der Formel IV 2,4-Dichlor-3-methylnitrobenzol, als Phenol der Formel VI Phenol oder 4-Fluorphenol und in der 2. Stufe als Amin der Formel III Ammoniak oder Methylamin verwendet wird.

4. Verfahren zur Herstellung von Verbindungen der Formel II durch Umsetzung eines Nitrobenzols der Formel

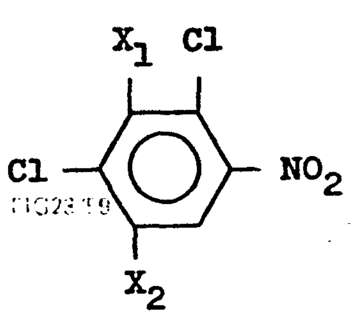

(IV),

in der $X_1$ und $X_2$ wie oben definiert sind, mit einem Phenol der Formel

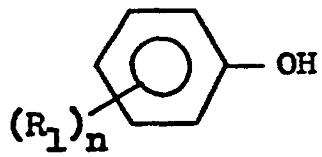

(VI),

FIG24/59

in der n und $R_1$ die obige Bedeutung haben, in Gegenwart einer wäßrigen Alkali- oder Erdalkalibase, dadurch gekennzeichnet, daß die Umsetzung in Gegenwart eines üblichen Phasentransferkatalysators zwischen 60 und 150°C erfolgt.

5. Verfahren nach Anspruch 4, dadurch gekennzeichnet, daß die Umsetzung bei Temperaturen zwischen 80 und 120°C erfolgt.

6. Verfahren zur Herstellung von Verbindungen der Formel I durch Umsetzung einer Diphenoxyverbindung der Formel

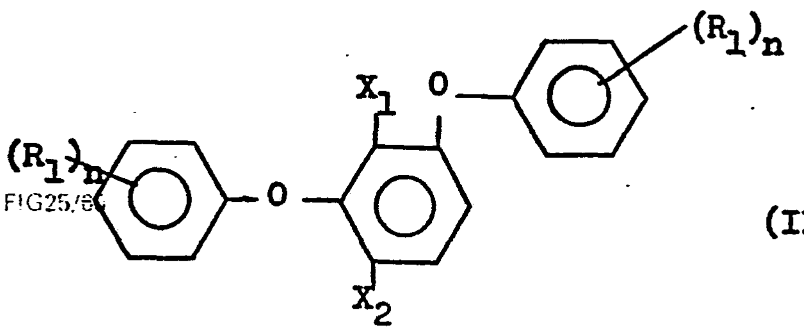

(II),

in der n, $R_1$, $X_1$ und $X_2$ die obige Bedeutung haben, mit einer Aminoverbindung der Formel

$H_2NR_2$ (III),

in der $R_2$ wie oben definiert ist, dadurch gekennzeichnet, daß die Umsetzung in einem Äthergruppierungen enthaltenden Alkohol bei Temperaturen zwischen 20 und 180°C erfolgt.

7. Verfahren nach Anspruch 6, dadurch gekennzeichnet, daß die Umsetzung bei Temperaturen zwischen 40 und 170°C erfolgt.

8. Verfahren nach Anspruch 4, dadurch gekennzeichnet, daß als Nitrobenzol der Formel IV 2,3,4-Trichlorbenzol und als Phenol der Formel VI Phenol oder 4-Fluorphenol verwendet wird.

9. Verfahren nach Anspruch 4, dadurch gekennzeichnet, daß als Nitrobenzol der Formel IV 2,4-Dichlor-3-methylnitrobenzol und als Phenol der Formel VI Phenol oder 4-Fluorphenol verwendet wird.

10. Verfahren nach Anspruch 1, dadurch gekennzeichnet, daß als Diphenoxyverbindung der Formel II eine Verbindung der Formel

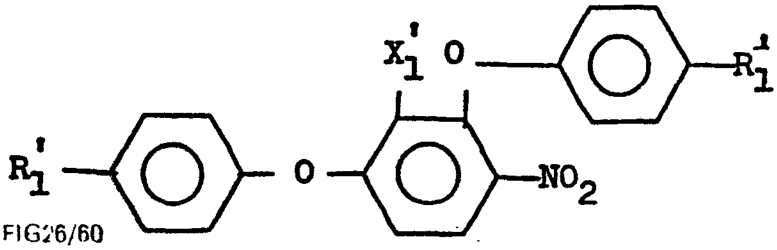

FIG26/60

(VII)

verwendet wird, in der $R'_1$ Wasserstoff oder Fluor, $X'_1$ Chlor oder Methyl bedeutet, und daß als Amin der Formel III Ammoniak oder Methylamin dient.

**Claims**

1. Process for the preparation of compounds of formula

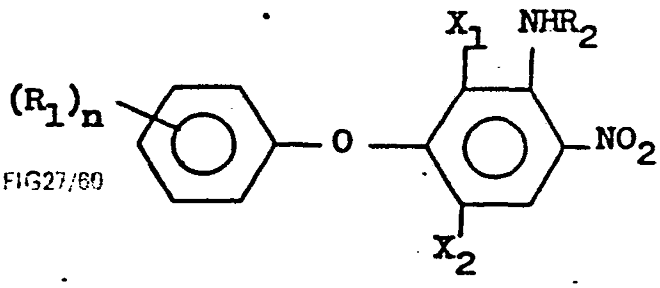

FIG27/60

(I)

wherein
n represents an integer from 1 to 3, if
$R_1$ represents hydrogen, halogen or alkyl with 1 to 8 carbon atoms
or
n represents 1 if
$R_1$ represents hydrogen, trifluoromethyl, alkoxy with 1 to 8 carbon atoms or acetyl,
$R_2$ represents hydrogen; alkyl, alkenyl or alkynyl with up to 16 carbon atoms; an alkyl group with 2 to 6 carbon atoms substituted by hydroxy, $C_{1-4}$ alkoxy, phenoxy, halophenoxy, $C_{1-4}$ alkylphenoxy, $C_{1-4}$ alkoxyphenoxy, nitrophenoxy, cyanophenoxy, amino or $C_{1-4}$ alkylthio; optionally halogen-substituted benzyl, $NR_3R_4$, $CHR_3$-$COOR_4$ or $CHR_3$-$CONR_4R_5$, whilst $R_3$, $R_4$ and $R_5$, which may be identical or different, represent hydrogen or $C_{1-8}$ alkyl groups, and
$X_1$ and $X_2$, which may be identical or different, represent hydrogen or halogen, and $X_1$ may also represent methyl,
wherein in a first step

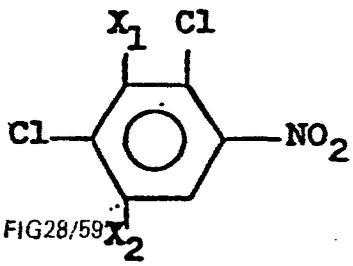

(IV)

wherein $X_1$ and $X_2$ are as hereinbefore defined, is reacted with a phenol of formula

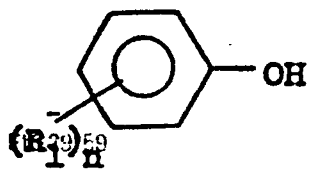

(VI)

wherein n and $R_1$ are as hereinbefore defined, in the presence of an aqueous alkali metal or alkaline earth metal base and in a second step the resulting compound of formula

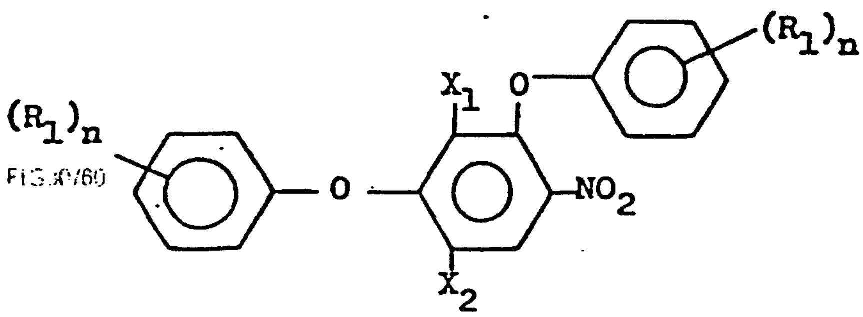

(II)

wherein n, $R_1$, $X_1$ and $X_2$ are as hereinbefore defined, is reacted with an amine of formula
$H_2NR_2$ (III)

wherein $R_2$ is as hereinbefore defined, characterised in that the first step of the reaction is effected in the presence of a conventional phase transfer catalyst and for the second step an alcohol which contains ether groupings is used as solvent.

2. Process as claimed in claim 1, characterised in that in the first step 2,3,4-trichloronitrobenzene is used as the nitrobenzene of formula IV and phenol or 4-fluorophenol is used as the phenol of formula VI and in the second step ammonia or methylamine is used as the amine of formula III.

3. Process as claimed in claim 1, characterised in that in the first step 2,4-dichloro-3-methylnitro-benzene is used as the nitrobenzene of formula IV, phenol or 4-fluorophenol is used as the phenol of formula VI and in the second step ammonia or methylamine is used as the amine of formula III.

4. Process for the preparation of compounds of formula II by reacting a nitrobenzene of formula

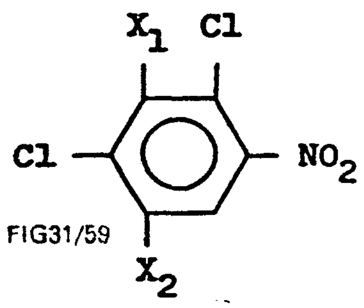

(IV)

wherein $X_1$ and $X_2$ are as hereinbefore defined, with a phenol of formula

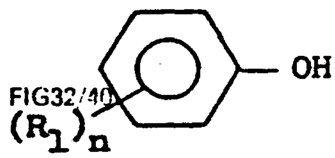

(VI)

wherein n and $R_1$ are as hereinbefore defined, in the presence of an aqueous alkali metal or alkaline earth metal base, characterised in that the reaction is carried out in the presence of a conventional phase transfer catalyst at between 60 and 150°C.

5. Process as claimed in claim 4, characterised in that the reaction is carried out at temperatures of between 80 and 120°C.

6. Process for preparing compounds of formula I by reacting a diphenoxy compound of formula

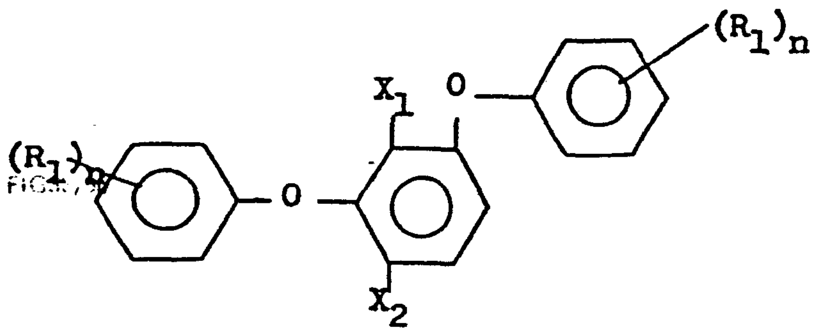

(II)

wherein n, $R_1$, $X_1$ and $X_2$ are as hereinbefore defined, with an amino compound of formula
$H_2NR_2$ (III)
wherein $R_2$ is as hereinbefore defined, characterised in that the reaction is carried out in an alcohol containing ether groupings at temperatures of between 20 and 180°C.

7. Process as claimed in claim 6, characterised in that the reaction is carried out at temperatures of between 40 and 170°C.

8. Process as claimed in claim 4, characterised in that 2,3,4-trichlorobenzene is used as the nitrobenzene of formula IV and phenol or 4-fluorophenol is used as the phenol of formula VI.

9. Process as claimed in claim 4, characterised in that 2,4-dichloro-3-methylnitrobenzene is used as the nitrobenzene of formula IV and phenol or 4-fluorophenol is used as the phenol of formula VI.

10. Process as claimed in claim 1, characterised in that a compound of formula

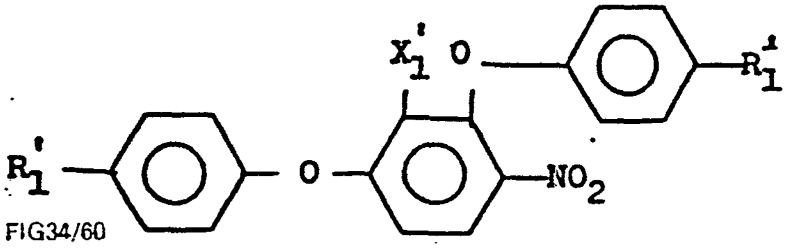

FIG34/60

(VII)

wherein $R_1'$ represents hydrogen or fluorine and $X'_1$ represents chlorine or methyl, is used as the diphenoxy compound of formula II and ammonia or methylamine as the amine of formula III.

**Revendications**

1. Procédé pour la préparation de composés de formule:

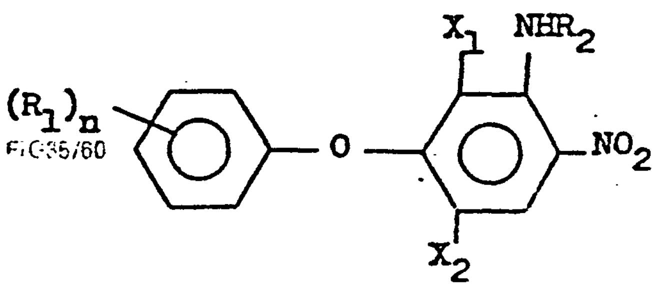

FIG35/60

(I)

dans laquelle:

n représente un nombre entier de 1 à 3, lorsque $R_1$ remplace hydrogène, halogène ou alcoyle avec 1 à 8 atomes de C

ou

n représente 1, lorsque

$R_1$ remplace hydrogène, trifluorométhyle, alcoxy avec 1 à 8 atomes de C, ou acétyle,

$R_2$ représente hydrogène; alcoyle, alcényle ou alcynyle avec jusqu'à 16 atomes de C; un radical alcoyle avec 2 à 6 atomes de C, substitué par hydroxy, alcoxy en $C_1$-$C_4$, phénoxy, halogénophénoxy, alcoyl(en $C_1$-$C_4$)phénoxy, alcoxy(en $C_1$-$C_4$)phénoxy, nitrophénoxy, cyanophénoxy, amino ou alcoylthio en $C_1$-$C_4$; benzyle, eventuellement halogéno substitué, $NR_3R_4$, $CHR_3$-$COOR_4$ ou $CHR_3$-$CONR_4R_5$, $R_3$,$R_4$ et $R_5$, qui peuvent être identiques ou différents, représentant hydrogène ou des radicaux alcoyle en $C_1$-$C_8$, et $X_1$ et $X_2$, qui peuvent être identiques ou différents, remplacent hydrogène ou halogène, $X_1$ pouvant représenter également méthyle,

selon lequel dans la première étape, un fait réagir:

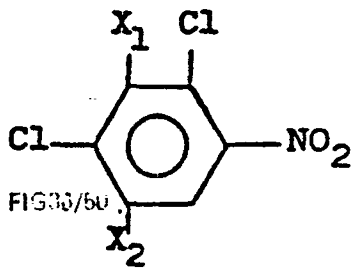 (IV),

dans lequel $X_1$ et $X_2$ ont la signification ci-dessus, avec un phénol de formule:.

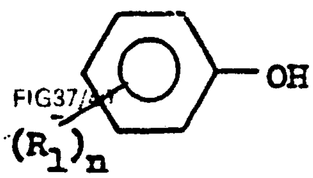 (VI),

dans laquelle n et $R_1$ ont la signification ci-dessus, en présence d'une base alcaline ou alcalino-terreuse aqueuse, et dans la deuxième étape, on fait réagir le composé obtenu de formule:.

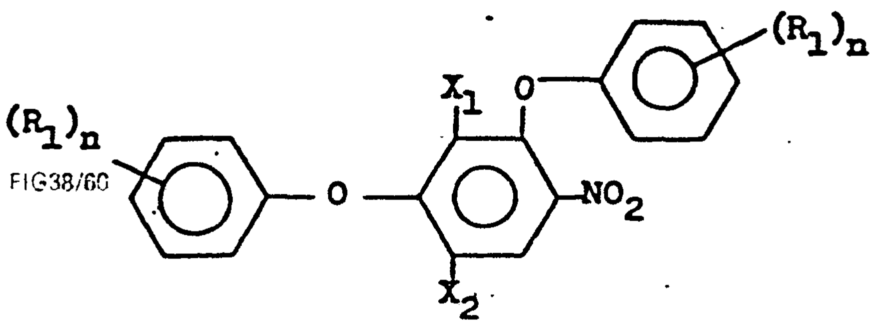 (II),

dans laquelle n, $R_1$, $X_1$ et $X_2$ ont la signification ci-dessus, avec une amine de formule: $H_2NR_2$ (III),

dans laquelle $R_2$ est défini comme plus haut, caractérisé en ce que la première étape de la réaction a lieu en presence d'un catalyseur de transfert de phases usuel et en ce que pour la deuxième étape, on utilise comme solvant un alcool contenant des groupements éther.

2. Procédé selon la revendication 1, caractérisé en ce que dans la première étape, on utilise en tant que nitrobenzène de formule IV, le 2,3,4-trichloronitrobenzène, en tant que phénol de formule VI, le phenol ou le 4-fluorophénol et dans la deuxième étape en tant qu'amine de formule III, l'ammoniac ou la méthylamine.

3. Procédé selon la revendication 1, caractérisé en ce que dans la première étape, on utilise en tant que nitrobenzène de formule IV,1e 2,4-dichloro-3-méthylnitro-benzène, en tant que phénol de formule VI, le phénol ou le 4-fluorophénol et dans la deuxième étape en tant qu'amine de formule III l'ammoniac ou la méthylamine.

4. Procédé pour la préparation de composés de formule II par réaction d'un nitrobenzène de formule:

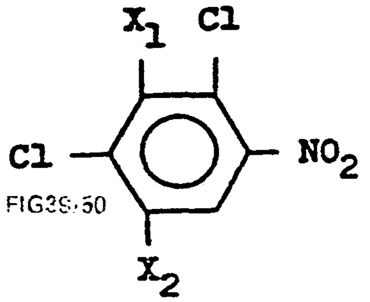

(IV),

dans laquelle $X_1$ et $X_2$ sont définis comme plus haut, avec un phénol de formule:

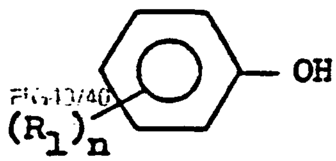

(VI).

dans laquelle n et $R_1$ ont la signification ci-dessus, en présence d'une base alcaline ou alcalino-terreuse aqueuse, caractérisé en ce que la réaction a lieu en présence d'un catalyseur de transfert de Phases usuel entre 60 et 150° C.

5. Procédé selon la revendication 4, caractérisé en ce que la réaction a lieu à des températures entre 80 et 120° C.

6. Procédé pour la préparation de composés de formule I par réaction d'un composé diphénoxy de formule:

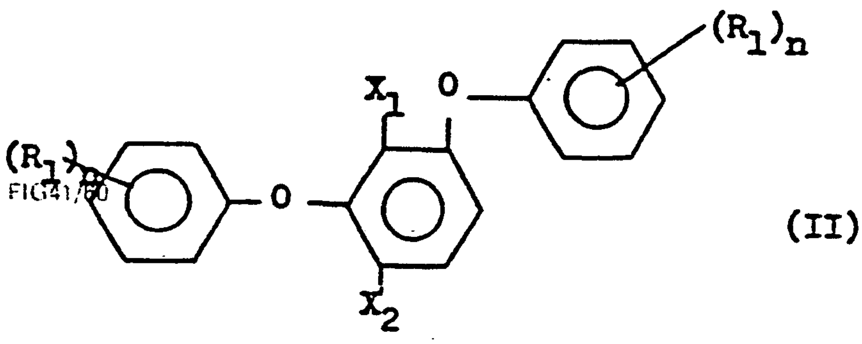

(II),

dans laquelle n, $R_1$, $X_1$ et $X_2$ ont la signification ci-dessus, avec un composé amino de formule:

$H_2NR_2$ (III),

dans laquelle $R_2$ est défini comme plus haut, caractérisé en ce que la réaction a lieu dans un alcool contenant des groupements éther à des températures entre 20 et 180° C.

7. Procédé selon la revendication 6, caractérisé en ce que la réaction a lieu à des températures entre 40 et 170° C.

8. Procédé selon la revendication 4, caractérisé en ce que l'on utilise, en tant que nitrobenzène de formule IV, le 2,3,4-trichlorobenzène et en tant que phénol de formule VI, le phénol ou le 4-fluorophénol.

9. Procédé selon la revendication 4, caractérisé en ce que l'on utilise en tant que nitrobenzène de formule IV le 2,4-dichloro-3-méthylnitrobenzène et en tant que phénol de formule VI le phénol ou le 4-fluorophénol.

10. Procédé selon la revendication 1, caractérisé en ce que l'on utilise comme composé diphenoxyde formule II, un composé de formule:

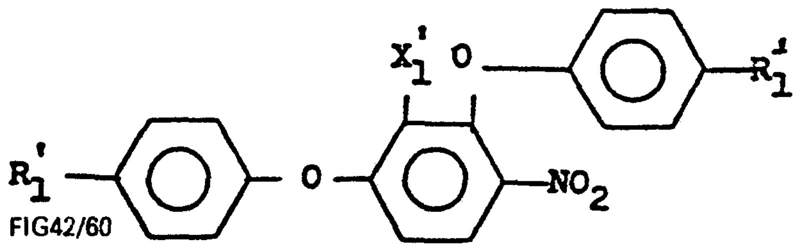

FIG42/60

(VII)

dans laquelle $R_1'$ représente hydrogène ou fluor, $X_1'$ représente chlore cu méthyle, et en ce que l'ammoniac ou la méthylamine sert d'amine de formule III.